(19) Europäisches Patentamt
European Patent Office
Office européen des brevets

(11) EP 1 658 839 A1

# (12) EUROPEAN PATENT APPLICATION

(43) Date of publication:
**24.05.2006 Bulletin 2006/21**

(21) Application number: **05090322.8**

(22) Date of filing: **21.11.2005**

(51) Int Cl.:
***A61K 9/127*** *(2006.01)* ***A61P 1/00*** *(2006.01)*
***A61P 37/06*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **19.11.2004 DE 102004056659**
**15.09.2005 EP 05020218**

(71) Applicant: **Novosom AG**
**06120 Halle (DE)**

(72) Inventors:
- **Panzner, Steffen**
  **06108 Halle (DE)**
- **Hecker, Markus**
  **69118 Heidelberg (DE)**
- **Endert, Gerold**
  **06114 Halle (DE)**
- **Fankhänel, Stefan**
  **01219 Dresden (DE)**

(74) Representative: **Ziebig, Marlene et al**
**Wallstrasse 58/59**
**10179 Berlin (DE)**

## (54) Oligonucleotide/carrier combinations targeting CD40

(57) To provide compositions for a topical treatment comprising inhibitory oligonucleotides directed against CD40 that have a therapeutic effect in vivo a pharmaceutical composition comprising an oligonucleotide as an active agent, which oligonucleotide is adapted to target nucleic acids encoding CD40 thereby to modulate the expression of CD40 in mammalian cells, and a liposome as an excipient; wherein said liposome is an amphoteric liposome, and a pharmaceutically acceptable carrier is proposed.

EP 1 658 839 A1

## Description

### Field of the invention

CD40 represents a key target in the treatment of inflammatory diseases such as Colitis Ulcerosa, Morbus Crohn, Rheumatoid Arthritis, Multiple Sclerosis, Lupus, Psoriasis, graft rejection and the like. The protein signals through its interaction with a trimeric ligand, CD154. As such, inhibition of the signalling event with small molecule inhibitors is unlikely and therapeutic developments have focused on the use of blocking antibodies directed against one of the two. Oligonucleotides directed against the mRNA of CD40 offer an alternative approach to interrupt the signalling cascade and a number of sequences have been validated in vitro so far. However, in vivo proof of concept has not been established so far and poor delivery of the active oligonucleotides is assumed to be the most likely reason. Carrier / drug combinations that allow a treatment of inflammatory diseases are provided. More specifically, formulations and administration protocols for topical administration are described.

### Definitions

Within that disclosure the following definitions should apply:

**Amphoter or amphoteric character**: A structure, being a substance or a mixture of substances or a supramolecular complex (e.g. a liposome) comprising charged groups of both anionic and cationic character wherein

(i) at least one of the charged groups has a pK between 4 and 8 and
(ii) the cationic charge prevails at pH 4 and
(iii) the anionic charge prevails at pH 8,

resulting in an isoelectric point of neutral net charge between pH4 and pH8. Amphoteric character by that definition is different from zwitterionic character, as zwitterions do not have a pK in the range mentioned above. In consequence, zwitterions are essentially neutrally charged over a range of pH values. Phosphatidylcholin or phosphytidylethanolamins are neutral lipids with zwitterionic character.

**Active drug** within the invention means one or more oligonucleotides, in particular one or more oligonucleotides directed against CD40.

**Excipient** within that invention means a liposome comprising one or more lipids, in particular an amphoteric liposome, but not comprising active drug or a specific vehicle.

**Vehicle** within that invention means a pharmaceutically acceptable carrier comprising water, buffer substances, salts, sugars, polymers and the like but not comprising active drug or excipient.

**Formulation** within that invention means the combination of active drug and excipient without specifying the vehicle.

**Composition** within that invention means the combination of active drug, excipient and vehicle.

### Background of the invention

CD40 was first described by Pauli et al. 1984 (Cancer Immunol. Immunotherapy 17: 173-179). The protein is primarily expressed on dendritic cells and B-cells and interacts with its ligand (CD40 ligand or CD154) on T-cells. The signalling between CD40 and CD154 is crucial for the development of a humoral immune response. Overstimulation of the pathway leads to immunological imbalance and consequently to a variety of immune associated disorders, including but not limited to graft rejection, graft-versus-host disease, multiple sclerosis, systemic lupus erythematosous, rheumatoid arthritis, asthma, inflammatory bowel disease, psoriasis, thyroiditis and others. CD40 overexpression might also be involved in tumor growth (Gruss et al. 1997, Leuk Lymphoma. 24(5-6):393-422). CD40 signals into the NF-κB pathway, consequently leading to the activation of the transcription factor and the eventual release of cytokines such as IL-1, TNFα or IFNγ, which in turn activate other cells, thus promoting the inflammation using a positive feedback mechanism. Inhibition of early events in that loop is recognized as an effective strategy to inhibit immune disorders or inflammation processes. Examples include the competitive binding of TNFα using antibodies, receptor blocking using antibodies against the TNFα-receptor, competitive inhibition of NF-κB binding and many more. More specifically, the CD40/ CD154 interaction can be blocked using antibodies targeted against one of the components, as described in Holstager et al.

2000 (J. Biol. Chem. 275: 15392-15398) or Baccam & Bishop 1999 (Eur. J. Immunol. 29: 3855-3866). However, side reactions of the CD40 antibodies under development ask for alternative means to cut the inflammatory feedback loop at this position.

Protein expression can very specifically be downregulated using oligonucleotides such as antisense, locked nucleic acids (LNA), peptide nucleic acids (PNA), morpholino nucleic acids (Morpholinos) or small interfering RNAs (siRNA) of various chemistries. US2004/0186071 and US6197584, both to Bennett et al. give a detailed description of such oligonucleotides based on antisense mechanisms. Pluvinet et al. in Blood, 2004 first described the down-regulation of CD40 using siRNA against the human target. Also, the WO2004/090108 to Manoharan describes the applicability of novel oligonucleotides to inhibit the expression of CD40 protein. Indirect means to downregulate the CD40 expression are described in DE10049549 to Hecker and Wagner, using the inhibition of transcription factor IFR-1.

It is known in the art, that oligonucleotides, irrespective of their actual chemical origin lack therapeutic efficacy due to instability in body fluids or inefficient uptake into cells or for both reasons. Chemical modifications of the oligonucleotide comprising but not limited to the abovementioned variants, also extended towards the formation of conjugates with ligands or polymers represent one strategy to overcome practical limitations. A second set of strategies involves the use of carrier systems, in particular liposomes for protection, targeting and enhanced uptake into cells. Such carrier system shall meet an optimum score of the following criteria: high encapsulation efficiency and economic production process, colloidal stability of the carrier, enhanced uptake into cells and of course low toxicity and immunogenicity. Anionic or neutral liposomes are often excellent in terms of colloidal stability, as no aggregation occurs between the carrier and the environment. Consequently their biodistribution is excellent and the potential for irritation and cytotoxicity is low. However, such carrier lack encapsulation efficiency and do not provide an endosomolytic signal that facilitates further uptake into cells (Journal of Pharmacology and experimental Therapeutics (2000), 292, 480-488 by Klimuk et al.).

A great many of publications deal with cationic liposomal systems, e.g. Molecular Membrane Biology (1999), 16, 129-140 by Maurer et al.; BBA (2000) 1464, 251-261 by Meidan et al.; Reviews in Biology and Biotechnology (2001), 1(2), 27-33 by Fiset & Gounni. Although cationic systems provide high loading efficiencies, they lack colloidal stability, in particular after contact with body fluids. Ionic interactions with proteins and/or other biopolymers lead to in situ aggregate formation with the extracellular matrix or with cell surfaces. Cationic lipids have often been found to be toxic as shown by Filion et al. in BBA (1997), 1329(2), 345-356; Dass in J. Pharm. Pharmacol. 2002), 54(5), 593-601; Hirko et al. in Curr. Med. Chem., 10(14), 1185-1193.

These limitations were overcome by the addition of components that provide a steric stabilization to the carriers. Polyethylenglycols of various chain length are known to eliminate the aggregation problems associated with the use of cationic components in body fluids and PEGylated cationic liposomes show enhanced circulation times in vivo (BBA (2001) 1510, 152-166 by Semple et al.). Still, the use of PEG does not solve the intrinsic toxicity problem associated with the cationic lipids. It is also known that PEG substantially inhibits the productive entry of such liposomes into the cells or their intracellular delivery (Song et al. in BBA (2002), 1558(1), 1-13).

Amphoteric liposomes represent a recently described class of liposomes having an anionic or neutral charge at pH7.5 and a cationic charge at pH4. Explicit reference is made here to WO 02/066490, WO 02/066012 and the WO 03/070735, all to Panzner et al. which give a detailed description of the amphoteric liposomes. Further disclosures are made in WO 03/070220 and WO 03 070735, also to Panzner et al. describing more pH sensitive lipids for the manufacturing of said amphoteric liposomes. Amphoteric liposomes have an excellent biodistribution and are very well tolerated in animals. They can encapsulate nucleic acid molecules with high efficiency.

In summary, CD40 clearly represents an attractive target for the treatment of inflammatory or immune disorders which potentially can be eliminated using oligonucleotide inhibitors such as antisense or siRNA molecules. However, there is no prior art showing the successful application of the active oligonucleotides in vivo. Current liposome technologies are not optimal to provide straightforward means for the formulation of oligonucleotides.

## Object of the invention

Therefore, the object of the invention described here is to provide compositions comprising inhibitory oligonucleotides directed against CD40 that have a therapeutic effect in vivo. A specific object of the invention is to provide compositions for a topical treatment.

## Summary of the invention

The present invention is directed to formulations comprising oligonucleotides and amphoteric liposomes, wherein said amphoteric liposomes are negatively or neutrally charged at pH 7.4 and cationic at pH4. A substantial portion or all of said oligonucleotides is physically entrapped into the amphoteric liposomes. In one preferred embodiment of the invention, the formulation is administered at a slightly acidic pH.

The invention further relates to the topical treatment of mammals or of parts of mammals, in particular humans or their organs suspected of having or being prone to a disease or condition associated with the expression of CD40 by administering a therapeutically or prophylactically effective amount of the liposomal formulations. Therefore, in a more specific application of the present invention compositions are used that target nucleic acids encoding CD40 and modulate the expression of CD40 in mammalian cells.

## Detailed description of the invention

Excipients of the present invention are amphoteric liposomes and comprise

a) a neutral phospholipid being either phosphatidylcholine or phosphatidylethanolamine or both
b) optionally cholesterol
c) an amphoteric lipid or alternatively a mix of lipid components with amphoteric properties.

Amphoteric lipids are disclosed in the WO 02/066489 as well as in the WO 03/070735. More preferably, the amphoteric lipids are selected from the group of HistChol, HistDG, isoHistSuccDG, Acylcarnosin and HCCHol.
Most preferred the amphoteric lipid is HistChol.
Amphoteric liposomes of the present invention may also be manufactured using pH-responsive anionic and/or cationic components, as disclosed in WO 02/066012. Cationic lipids sensitive to pH are disclosed in WO 02/066489 A2/A3 and WO 03/070220 A1 and in the references made therein, in particular in Budker et al. 1996, Nat Biotechnol. 14(6):760-4 and can be used in combination with constitutively charged anionic lipids or with anionic lipids that are sensitive to pH. Vice versa, the cationic charge may also be introduced from constitutively charged lipids that are known to those skilled in the art in combination with a pH sensitive anionic lipid. Combinations of constitutively charged anionic and cationic lipids, e.g. DOTAP and DPPG are not preferred.
Preferred cationic components are selected from the group of DPIM, CHIM, DORIE, DDAB, DAC-Chol, TC-Chol, DOTMA, DOGS, $(C18)_2Gly^+$ N,N-dioctadecylamido-glycin, CTAB, CPyC, DODAP and DOEPC.
Further preferred cationic lipids are DMTAP, DPTAP, DOTAP, DC-Chol, MoChol and HisChol.
The amphoteric mixtures further comprise anionic lipids, either constitutively or conditionally charged in response to pH and those lipids are also known to those skilled in the art. Preferred lipids for use with the invention are selected from the group comprising: DOGSucc, POGSucc, DMGSucc, DPGSucc, DMPS, DPPS, DOPS, POPS, DMPG, DPPG, DOPG, POPG, DMPA, DPPA, DOPA, POPA, CHEMS and CetylP.
Further preferred anionic lipids are DOGSucc, DMGSucc, DMPG, DPPG, DOPG, POPG, DMPA, DPPA, DOPA, POPA, CHEMS and CetylP.

Neutral phosphatidylcholins or phosphoethanolamins or mixtures of the two are also present in the mixture to the molar amount not occupied by the former two groups, but to at least 20mol%. Preferred components are selected from the group of DMPC, DPPC, DSPC, POPC, DOPC or from phosphatidylcholins from natural sources such as soy bean PC or egg PC. Preferred phosphatidylethanolamins are selected from DOPE or DMPE or DPPE.
Most preferred neutral lipids are DOPE and POPC.

Abbreviations for lipids refer primarily to standard use in the literature and are included here as a helpful reference:

| | |
|---|---|
| DMPC | Dimyristoylphosphatidylcholin |
| DPPC | Dipalmitoylphosphatidylcholin |
| DSPC | Distearoylphosphatidylcholin |
| POPC | Palmitoyl-oleoylphosphatidylcholin |
| DOPC | Dioleoylphosphatidylcholin |
| DOPG | Dioleoylphosphatidylglycerol |
| POPG | Palmitoyl-oleoylphosphatidylglycerol |
| DMPG | Dimyristoylphosphatidylglycerol |
| DPPG | Dipalmitoylphosphatidylglycerol |
| DMPS | Dimyristoylphosphatidylserin |
| DPPS | Dipalmitoylphosphatidylserin |
| DOPS | Dioleoylphosphatidylserin |
| POPS | Palmitoyl-oleoylphosphatidylserin |
| DMPA | Dimyristoylphosphatidic acid |
| DPPA | Dipalmitoylphosphatidic acid |
| DOPA | Dioleoylphosphatidic acid |
| POPA | Palmitoyl-oleoylphosphatidic acid |
| Chems | Cholesterolhemisuccinat |

| | |
|---|---|
| DC | -Chol 3-β-[N-(N',N'-dimethylethane) carbamoyl]cholesterol |
| CetylP | Cetylphosphat |
| DODAP | (1,2)-dioleoyloxypropyl)-N,N-dimethylammonium chloride |
| DOEPC | 1,2-dioleoyl-sn-glycero-3-ethylphosphocholin |
| DAC-Chol | 3-β-[N-(N,N'-dimethylethane) carbamoyl]cholesterol |
| TC-Chol | 3-β-[N-(N',N', N'-trimethylaminoethane) carbamoyl] cholesterol |
| DOTMA | (1,2-dioleyloxypropyl)-N,N,N-trimethylammoniumchlorid) (Lipofectin®) |
| DOGS | ((C18)$_2$GlySper3$^+$) N,N-dioctadecylamido-glycyl-spermin (Transfectam®) CTAB Cetyl-trimethylammoniumbromid, |
| CPyC | Cetyl-pyridiniumchlorid |
| DOTAP | (1,2-dioleoyloxypropyl)-N,N,N-trimethylammonium Salz |
| DMTAP | (1,2-dimyristoyloxypropyl)-N,N,N-trimethylammonium Salz |
| DPTAP | (1,2-dipalmitoyloxypropyl)-N,N,N-trimethylammonium Salz |
| DOTMA | (1,2-dioleyloxypropyl)-N,N,N-trimethylammonium chlorid) |
| DORIE | (1,2-dioleyloxypropyl)-3 dimethylhydroxyethyl ammoniumbromid) |
| DDAB | Dimethyldioctadecylammonium bromid |
| DPIM | 4-(2,3-bis-palmitoyloxy-propyl)-1-methyl-1H-imidazole |
| CHIM | Histaminyl-Cholesterolcarbamat |
| MoChol | 4-(2-Aminoethyl)-Morpholino-Cholesterolhemisuccinat |
| HisChol | Histaminyl-Cholesterolhemisuccinat. |
| HCCho | l Nα-Histidinyl-Cholesterolcarbamat |
| HistChol | Nα-Histidinyl-Cholesterol-hemisuccinat. |
| AC | Acylcarnosin, Stearyl- & Palmitoylcarnosin |
| HistDG | 1,2—Dipalmitoylglycerol-hemisuccinat-Nα-Histidinyl-hemisuccinat, & Distearoyl- ,Dimyristoyl, Dioleoyl or palmitoyl-oleoylderivatives |
| IsoHistSuccDG | 1,2—Dipalmitoylglycerol-Oα-Histidinyl-Nα-hemisuccinat, & Distearoyl-, Dimyristoyl, Dioleoyl or palmitoyl-oleoylderivatives |
| DGSucc | 1,2—Dipalmitoyglycerol-3-hemisuccinat & Distearoyl-, dimyristoyl-Dioleoyl or palmitoyl-oleoylderivatives |

The following table provides non-limiting examples of lipids that are suitable for use in the compositions in accordance with the present invention. The membrane anchors of the lipids are shown exemplarily and serve only to illustrate the lipids of the invention and are not intended to limit the same.

| | |
|---|---|
| MoChol 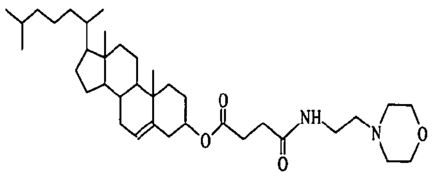 | DG-Succ 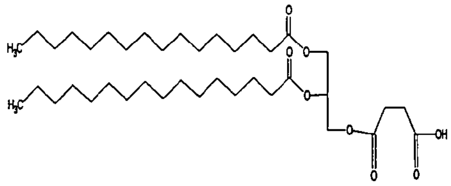 |
| DOTAP 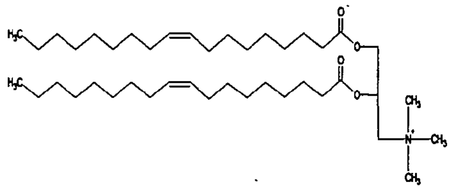 | IsohistsuccDG 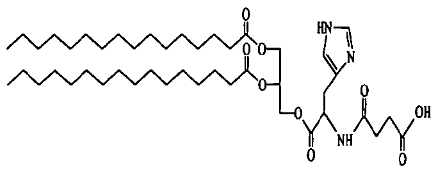 |
| HisChol 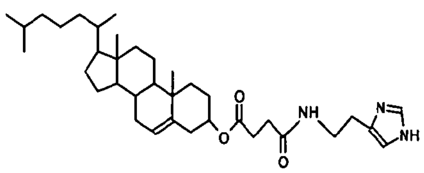 | HCChol 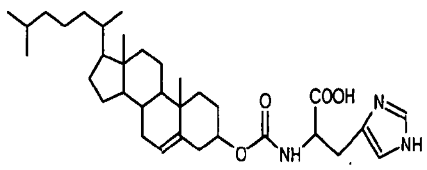 |

| | |
|---|---|
| AC 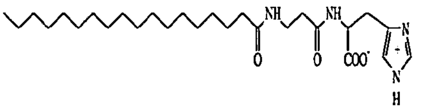 | |
| Hist-Chol 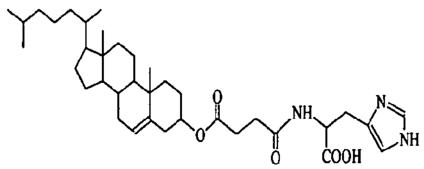 | |
| Hist-DG 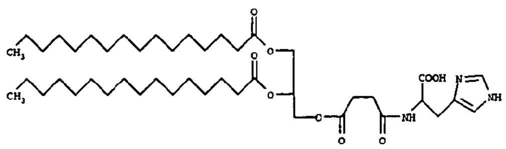 | |

Drugs of the current invention are oligonucleotides targeting nucleic acids encoding CD40 and thereby modulating the expression of CD40. The term "target nucleic acid" or the term "nucleic acids encoding CD40" encompass DNA encoding for CD40 as well as all RNAs derived from such DNA, being pre-mRNA or mRNA. A specific hybridisation between the target nucleic acid and on or more oligonucleotides directed against such sequences result in an inhibition of CD40 expression. To achieve such specific targeting, the oligonucleotide must comprise a continuous stretch of nucleotides being complementary to the sequence of the target nucleic acid. Such oligonucleotides may vary in length between as little as 10, preferably 15 and even more preferably 18 and 50, preferably 30 and more preferably 25 nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one mismatch within the said continuous stretch of base pairs, although this is less preferred.

Oligonucleotides fulfilling the abovementioned criteria may be built with a number of different chemistries and topologies. Oligonucleotides may be single stranded or double stranded. Single stranded oligonucleotides include but are not limited to DNA-based oligonucleotides, locked nucleic acids, 2'-modified oligonucleotides and others, commonly known as antisense oligonucleotides. Backbone or base modifications may include but are not limited to Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, blockcopolymers or gapmers or in other arrangements.

In addition to the aforementioned oligonucleotides, the CD40 expression can also be inhibited using double stranded RNA molecules containing the complementary sequence motifs. Such RNA molecules are known as siRNA molecules in the art. Again, various chemistries were adapted to this class of oligonucleotides. Also, DNA /RNA hybrid systems are known in the art.

More specifically, explicit reference is made here to the US 6,197,584 and the US 2004/0186071, both to Bennett, describing in detail useful sequences and chemistries of such oligonucleotides. Reference is also made to Pluvinet et al. in Blood, 2004, describing siRNA sequence motifs for the inhibition of CD40. Further siRNA motifs are in public domain and can be purchased, e.g. from Santa Cruz Biotechnology (Santa Cruz, U.S.A.).

In one embodiment of the present invention, the formulations can be applied at a physiological pH between 7 and 8.

In one preferred embodiment of the present invention, the formulations can be applied at a slightly acidic pH, in particular at a pH below the isoelectric point of the respective excipient. More preferred, the pH of the composition is not lower than pH3.5 and most preferred the composition has a pH between 4 and 5 when applied.

Pharmaceutically acceptable vehicles for such application are known to those skilled in the art and include but are not limited to water, saline or phosphate buffered saline and the like for compositions having a neutral pH.

As low pH is detrimental to the long-term stability of the used oligonucleotides and /or lipids, the pH is preferentially adjusted to the lower value before use. Means to achieve this under pharmacologically acceptable standards are known to those of ordinary skill in the art and include but are not limited to mixing the storage stable colloid with an appropriate amount of acetic acid, citric acid or glycine, preferentially buffered to a lower pH, more preferred buffer between pH2 and pH4.

Lyophilization of the formulation provides further means for stabilization. In one preferred embodiment of the present invention the composition is lyophilized at the abovementioned acidic pH and reconstituted with water for injection prior to use. If lyophilization is part of the manufacturing procedure, protecting agents such as sugars or amino acids or polymers can be present in the vehicle.

Methods for the manufacturing of formulations are known to those skilled in the art. They include but are not limited to extrusion through membranes of defined pore size, injection of lipid solutions in ethanol into the water phase containing cargo or high pressure homogenization.

Also, it is known in the art that oligonucleotides can be contacted with the excipient at neutral pH, resulting in volume inclusion of a certain percentage of the solution containing the oligonucleotide. High concentrations of excipients ranging from 50mM to 150mM are preferred to achieve substantial encapsulation of the drug.

In contrast to such standard procedure, amphoteric liposomes offer the distinct advantage of binding oligonucleotides at or below their isoelectric point and thereby concentrating the drug at the liposome surface. Such process is described in WO 02/066012 A2/A3 in more detail.

Irrespective of the actual production process the non-encapsulated oligonucleotide can be removed from the liposomes after the initial production step wherein liposomes are formed as tight containers. Again, the technical literature and the references included here describe such methodology in detail and suitable process steps may include but are not limited to size exclusion chromatography, sedimentation, dialysis, ultrafiltration or diafiltration and the like.

However, such removal of non-encapsulated material is not mandatory and in one preferred embodiment of the invention the formulation comprises entrapped as well as free drug.

The particle size of the formulation is between 50 and 500nm, more preferred between 100 and 500nm and even more preferred between 150 and 300nm.

Some combinations of process steps are used with specific advantage:

(A)

- encapsulation of drug at neutral pH using drug concentrations between 0,5mg/mL and 50mg/mL, more preferred between 1 and 20mg/mL and excipient concentrations between 50mM and 150mM.
- vehicle can be water, saline or buffered saline
- actual liposome formation and sizing step
- non- entrapped drug not removed
- optional lyophilization and reconstitution with water
- storage form suspension or lyophilized powder
- administration at neutral pH

(B)

- encapsulation of drug at neutral pH using drug concentrations between 0,5mg/mL and 50mg/mL, more preferred between 1 and 20mg/mL and excipient concentrations between 50mM and 150mM.
- vehicle can be water, saline or buffered saline
- actual liposome formation and sizing step
- non- entrapped drug removed
- storage form suspension
- administration at neutral pH

(C)

- encapsulation of drug at neutral pH using drug concentrations between 0,5mg/mL and 50mg/mL, more preferred between 1 and 20mg/mL and excipient concentrations between 50mM and 150mM.
- vehicle can be water, saline or buffered saline
- actual liposome formation and sizing step
- non- entrapped drug removed
- storage form suspension
- pH is adjusted below the isoelectric point of the respective excipient
- administration at acidic pH

(D)

- encapsulation of drug at neutral pH using drug concentrations between 0,5mg/mL and 50mg/mL, more preferred between 1 and 20mg/mL and excipient concentrations between 50mM and 150mM.
- vehicle can be water, saline or buffered saline
- actual liposome formation and sizing step
- non- entrapped drug removed
- pH is adjusted below the isoelectric point of the respective excipient and addition of protectants
- lyophilization
- storage form: powder
- reconstitution and administration at acidic pH

(E)

- encapsulation of drug at a pH below the isoelectric point of excipient using a molar ratio of cationic charges of the excipient to anionic charges of the drug between 2 and 20, more preferred between 2,5 and 10 and even more preferred between 3 and 5 and excipient concentrations between 0,2mM and 100mM, more preferred between 0,5mM and 20mM and even more preferred between 1mM and 10mM.
- vehicle can be buffered with acetic acid, citric acid and the like and may further contain sodium chloride or sucrose.
- actual liposome formation and sizing step
- addition of cryoprotectants and lyophilization
- storage form: powder
- reconstitution and administration at acidic pH

(F)

- encapsulation of drug at a pH below the isoelectric point of excipient using a molar ratio of cationic charges of the excipient to anionic charges of the drug between 2 and 20, more preferred between 2,5 and 10 and even more preferred between 3 and 5 and excipient concentrations between 1mM and 100mM, more preferred between 2mM and 50mM and even more preferred between 5mM and 20mM.
- vehicle can be buffered with acetic acid, citric acid and the like and may further contain sodium chloride or sucrose.
- actual liposome formation and sizing step
- raise pH to neutrality
- select a combination of further process steps from (A), (B), (C) or (D).

The present invention describes pharmaceutical composition comprising oligonucleotides directed against CD40 for topical treatment. Without being limited to the examples given here, such formulations are therapeutically active in the treatment of inflammatory bowel disease and graft rejection. The use of the formulation is therefore for the prevention or treatment of inflammations, immune or autoimmune disorders, including but not limited to graft rejection, graft-versus-host disease, inflammatory bowel disease, Morbus Crohn, Colitis ulcerosa, Asthma bronchiale, COPD, atopic dermatitis, psoriasis, allergic rhinitis and the like.

Also, reasonable application of said formulations is believed to be within the skill of those in the art. Dosing is dependent on severity and responsiveness of the disease state treated, with the course of treatment lasting from several days to several month, or until cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of the individual oligonucleotides in the composition and can generally be estimated based on EC50 values found to be effective in animal models. In general, the dosage is from 0.01μg to 5mg oligonucleotide of kg body weight and may be given daily, weekly, monthly or yearly or even less regular. Persons of ordinary skill in the art can easily estimate repetition rates for dosing based on measured residence times and concentrations of the drug in body fluids or tissues. Following successful treatment, it may be desirable to have the patient undergo maintainance therapy to prevent the recurrence of the disease state, wherein the formulation is administered at maintenance doses, ranging from 0.01μg to 5mg oligonucleotide per kg of body weight, once or more daily to once in a year. While the present invention has been described with specificity in accordance with certain of its preferred embodiments, the following examples serve only to illustrate the invention and are not intended to limit the same.

**Example 1: Preparation of CD40-ODN-containing liposomes**

A mixture of 85 μmol POPC, 42 μmol CHEMS and 14 μmol DOTAP was dissolved in chloroform and evaporated in a round bottom flask to dryness under vacuum.
ODN with the sequence T*C*C*TAGATGGACCGCT*G*T was used with asterisks indicating a phosphorothioate linkage between the nucleotides (after Gao, Ph.D. thesis, Goettingen 2003, rAS3).
Lipid films were hydrated with 1 mg ODN in 1mL of buffer (10mM sodium acetate, 150mM NaCl pH4.5). The suspensions were hydrated for 25 minutes in a water bath at room temperature, sonicated for 5 minutes and eventually frozen at -70°C. After thawing the liposomal suspensions were extruded 15 times through polycarbonate membranes with a pore size of 400nm. The liposome suspensions were brought to pH 7.5 using 1M HEPES buffer and to 0.8M sucrose using a stock solution. Nonencapsulated ODN was removed from the extruded sample by flotation through 0.5M sucrose overlaid with 10mM HEPES, 150mM NaCL pH7.5 and the liposome suspension was stored at 4°C. Resulting liposomes were characterized by dynamic light scattering and found to be 220 to 250 nm in size.

**Example 2: Colitis induction**

Colitis was induced by using a single intra-colonic application of 2,4,6-trinitrobenzene sulphonc acid (TNBS) prepared by adding 20mg of TNBS to 135μl of 35%ethanol in 150mM NaCl. Male Wistar rats (200...250g) were placed under light ether anaesthesia and the mixture was administered using an 8cm long catheder inserted through the anal canal into the descending colon. After removing the catheder, rats were held in a headfirst position for 30s to avoid flowing out of the enema and rats were kept under normal condition afterwards.

**Example 3: Treatment and analysis**

Rats were treated with CD40 antisense from example 1 either 4 hours before or 3 days after the colitis induction. The antisense suspension from example 1 was brought to pH 4.5 using 1M buffered acetic acid/ sodium acetate pH 4.0 and a total of 100μl containing 2,7μg CD40 antisense suspension was applied to the colon according to example 2.
Seven days after induction of the colitis the animals were sacrificed. The colon was removed and opened longitudinally. Colon samples were fixed in PBS containing 4% formaldehyde. Paraffin-embedded sections (5μm) were stained with haematoxylin/eosin followed by microscopic inspection.

Colonic damage was scored according to the following criteria:

Table 1. Criteria for microscopic scoring of colonic damage.

| *Parameters* | Score |
|---|---|
| *Ulceration* | |
| No | 0 |
| Minor | 1 |
| Major | 2 |
| *Inflammation* | |
| None | 0 |
| Minor | 1 |
| Major | 2 |
| Severe | 3 |

Table continued

| | |
|---|---|
| *Depth of lesion* | |
| None | 0 |
| Superficial | 1 |
| One third | 2 |
| Two third | 3 |
| Transmural | 4 |
| *Fibrosis* | |
| None | 0 |
| Minor | 1 |
| Major | 2 |
| *Lymphocyte infiltration* | |
| No | 0 |
| Yes | 1 |
| Total score | 0 - 12 |

Results are presented in the figures 1 to 3 and demonstrate a very substantial reduction of the experimental colitis when treated with antisense directed against CD40, but not with the scrambled control antisense. Quite surprisingly, even a single treatment of a fully developed colitis at day 3 resulted in a strong and almost complete reduction of the inflammation. In confirmation to that, prevention of the colitis was also achieved when the formulation was applied in a preventive mode before the initiation of the disease.

**Example 4 - Alternative Formulation**

When used as excipient, a mixture of 60mol% POPC, 20mol% HistChol and 20mol% Cholesterol also resulted in successful treatment of the experimental colitis.

**Example 5 - Non removal of outside Antisense**

When used as a formulation, non-removal of non encapsulated antisense also resulted in carrier systems that are stable colloids.

**Example 6 - Small bowel transplant treatment**

Heterotopic small bowel transplantation was performed in male rats in the allogeneic Brown Norway (RT1 n) to Lewis (RT1l) strain combination without immunosuppressant therapy.
After explantation of the small bowel and flushing of the graft vessels with Ringer solution, one group of animals (n=3) received donor small bowel transplants pre-treated with the CD40 antisense ODN (group A) or the corresponding scrambled control ODN (group B) formulated in excipient as described in example 1. The antisense suspension from example 1 was brought to pH 4.5 using 1M buffered acetic acid/ sodium acetate pH 4.0.
Donor blood vessels were pre-treated with 2 ml Ringer solution containing CD40 antisense or scrambeled control ODN (2.7 μg DNA in a total volume of 100 μl). The bowel lumen was rinsed with UW (University of Wisconsin) solution. After 2 hours of cold ischemia, the DNA solution was flushed out and the grafts were implanted and analyzed histologically.

**Analysis**

To characterize overall mucosal perfusion, a perfusion index (PI) was calculated by using the equation

$$PI (\%) = (Vp + 0,5 \times Vip)/Vt$$

where Vp represents the number of perfused villi, Vip the number of all irregularly perfused villi and Vt the total number of villi observed.
To characterize overall mucosal perfusion damage, a stasis index (SI) was calculated as follows:

$$SI\ (\%) = Vnp/Vt$$

where Vnp represents the number of non-perfused villi and Vt the total number of all villi observed.

Further analysis of microcirculatory parameters in the mucosa and muscle layers included the assessment of functional capillary density (FCD, length of perfused capillaries per villus area (1/cm) at a magnification of 476x) and red blood cell velocity (RBCV in mm/sec at a magnification of 933x). Analysis of functional capillary density and red blood cell velocity were performed by using the CAPIMAGE software (Zeintl, Heidelberg, Germany), red blood cell velocity was determined by line-to-shift analysis. Furthermore, by using the fluorescent marker Rhodamine 6G adherent leukocytes were identified in each vessel segment (100 μm) and counted as cells that did not move or detach from the endothelium within an observation period of 30 s. Their number was calculated from the diameter and length of the blood vessel, assuming a cylindrical geometry, and expressed as number of cells per $mm^2$.

**Results**

**Microcapillary perfusion of villi within the graft mucosa**

Overall villi perfusion in the graft mucosa was significantly improved in CD40 antisense ODN-treated transplants as compared to the untreated control or scrambeled control ODN, respectively. This was shown by the perfusion index representing the percentage of perfused villi in respect to the observed villi per observation field (Figure 4A). Conversely the stasis index, a marker of the percentage of non-perfused villi in respect to the total number of villi per observation field, was significantly reduced in CD40 antisense ODN treated grafts compared to the untreated control and scrambeled control ODN, respectively (Figure 4A).

A more detailed analysis of single villus perfusion by measuring the functional capillary density within a single villus per villus area showed again a significantly higher density of perfused capillaries in CD40 antisense ODN treated animals compared to the untreated control and scrambeled control ODN, respectively (Figure 4B). This demonstrates a better preserved villus perfusion and hence better mucosal function CD40 antisense ODN treated animals.

Accordingly, measuring red blood cell velocity in the villus capillaries revealed a significantly greater velocity in CD40 antisense ODN treated transplants compared to the untreated control and scrambeled control ODN, respectively (Figure 4C).

**Leukocyte-endothelial cell interaction**

In contrast, evaluation of leukocyte-endothelial cell interaction in submucosal postcapillary venules revealed no significant differences in the number of sticking leukocytes to the endothelial surface between the different treatment and control groups (Fig. 4D).

**Example 7 - Materials**

This example provides non-limiting examples of CD40 nucleotide sequences that may be targeted by oligonucleotides that modulate the expression of CD40 and that are suitable for use in the compositions in accordance with the present invention.

Human CD40 mRNA (GenBank accession no. X60592)

Human CD40 mRNA sequence for targeting in accordance with the present invention is presented in SEQ ID NO:1. Related sequence information is found in published patent application number US 2004/0186071 (i.e., SEQ ID NO:85) to Bennett, et al. and in US patent no. 6197584 (i.e., SEQ ID NO:85) to Bennett, et al. and in Pluvinet, et al., *Blood,* 2004, 104(12), 3642-3646, the contents of which are incorporated by reference herein.

(SEQ ID NO:1):

```
1   gcctcgctcg ggcgcccagt ggtcctgccg cctggtctca cctcgccatg gttcgtctgc
61  ctctgcagtg cgtcctctgg ggctgcttgc tgaccgctgt ccatccagaa ccacccactg
121 catgcagaga aaaacagtac ctaataaaca gtcagtgctg ttctttgtgc cagccaggac
181 agaaactggt gagtgactgc acagagttca ctgaaacgga atgccttcct tgcggtgaaa
241 gcgaattcct agacacctgg aacagagaga cacactgcca ccagcacaaa tactgcgacc
301 ccaacctagg gcttcgggtc cagcagaagg gcacctcaga aacagacacc atctgcacct
361 gtgaagaagg ctggcactgt acgagtgagg cctgtgagag ctgtgtcctg caccgctcat
421 gctcgcccgg ctttggggtc aagcagattg ctacaggggt ttctgatacc atctgcgagc
481 cctgcccagt cggcttcttc tccaatgtgt catctgcttt cgaaaaatgt cacccttgga
541 caagctgtga gaccaaagac ctggttgtgc aacaggcagg cacaaacaag actgatgttg
601 tctgtggtcc ccaggatcgg ctgagagccc tggtggtgat ccccatcatc ttcgggatcc
661 tgtttgccat cctcttggtg ctggtcttta tcaaaaaggt ggccaagaag ccaaccaata
721 aggcccccca ccccaagcag gaaccccagg agatcaattt tcccgacgat cttcctggct
781 ccaacactgc tgctccagtg caggagactt tacatggatg ccaaccggtc acccaggagg
841 atggcaaaga gagtcgcatc tcagtgcagg agagacagtg aggctgcacc cacccaggag
901 tgtggccacg tgggcaaaca ggcagttggc cagagagcct ggtgctgctg ctgcaggggt
961 gcaggcagaa gcggggagct atgcccagtc agtgccagcc cctc
```

Mus musculus CD40 mRNA

Murine CD40 mRNA sequence for targeting in accordance with the present invention is presented in SEQ ID NO:2. Related sequence information is found in published patent application number US 2004/0186071 (i.e. SEQ ID NO:132) to Bennett, et al., the contents of which are incorporated by reference herein.

(SEQ ID NO:2):

```
gcctcctggc ccttcagctg tggtctttcc cgttttctga ctttgcggtg acactgggga     60
cttccttaga cctctctgga gacgctttcg gttctgcaga gattcccagg ggtattgtgg    120
gtggggtggg gtaacaatag tgtccctgtg gcgctcccag tccctatagt aatccttcac    180
ccctctgcta tcttgcaatc aggagagtcc ttagccctgc tataggtggc ttttgaggtc    240
ctggatgcga ggagggggac tggggggtgg gtcgggtaat gtaagaaaag ggctcctttt    300
gggaccctgg ctcctccagc caccttggtg cccatccctt aaactcttgg ggacaatcag    360
actcctggga aggtcctggg gaaatccctg ctcagtgact agccataggc ccaccgcgat    420
tggtgcccga agaccccgcc ctcttcctgg gcgggactcc tagcagggac tttggagtga    480
cttgtggctt cagcaggagc cctgtgattt ggctcttctg atctcgccct gcgatggtgt    540
ctttgcctcg gctgtgcgcg ctatggggct gcttgttgac agcggtgagt ggcttgtgtt    600
ctaacctcca agggagttag ggcttagaga gtgagagatg gaaagaggaa agaggagaca    660
agactttgga gatgagagat cttcctactg gaagcggcgg ttagtaggat gggcaagatc    720
tctcgcgtct tgacacacac acacacacac acaaatgagg tgggctgctc ctctttcctt    780
ccagaaggtc ggggttctgt tccacgaagc ccacagggaa ccttagggag ggcattcctc    840
cacagcggtg cctggacagc tttgtctgac ccaagccttg ctccggagct gactgcagag    900
actggaaagg gttagcagac aggaagcctg gctggggg                            938
```

Rat CD40 mRNA (GenBank accession no. AF 241231)

Rat CD40 mRNA sequence for targeting in accordance with the present invention is presented in SEQ ID NO: 3. (See, Gao, Ph.D. thesis, Goettingen 2003).

(SEQ ID NO:3):

```
1   tgggacccct gtgatctggc tgctctgatc tcgctctgca atgctgcctt tgcctcagct
61  gtgcgcgctc tggggctgct tgttgacagc ggtccatcta ggacagtgtg ttacgtgcag
121 tgacaaacag tacctccaag gtggcgagtg ctgcgatttg tgccagccgg gaaaccgact
181 agttagccac tgcacagctc ttgagaagac ccaatgccaa ccgtgcgact caggcgaatt
241 ctcagctcac tggaacaggg agatccgctg ccaccagcac cgacactgcg aactcaatca
301 agggcttcag gttaagaagg agggcaccgc ggtntcagac actgtttgta cctgcaagga
361 agggcagcac tgcgccagca aggagtgcga gacgtgcgct cagcacaggc cctgtggccc
421 tggctttgga gtcgtgcaga tggccactga gactactgat accgtctgcc aaccctgccc
481 ggtcggattc ttctccaatg ggtcatcact ttttgaaaag tgtcatccat ggacaagctg
541 tgaagat
```

Porcine CD40 cDNA

Porcine CD40 cDNA sequence for targeting in accordance with the present invention is presented in SEQ ID NO:4. (FIG. 7). Related sequence information is found in Rushworth, et al., *Transplantation,* 2002, 73(4), 635-642, the contents of which are incorporated by reference herein.

In addition, the following provide non-limiting examples of anti-CD40 oligonucleotides, e.g., antisense CD40 nucleic acid sequences, that are suitable for use in the present invention:

Oligonucleotides against human CD40

Examples of human antisense CD40 oligonucleotides are presented below. Further sequence information is found in published patent application number US 2004/0186071 and US Patent No. 6197584 to Bennett, et al., the contents of which are provided by reference herein. The SEQ ID NOs referred to by Bennett, et al. are provided to the right.

| | | | | |
|---|---|---|---|---|
| SEQ ID NO: | 5 | ccaggcggca ggaccact | Seq ID No: 1 | of Bennett et al. |
| SEQ ID NO: | 6 | gaccaggcgg caggacca | Seq ID No.:2 | of Bennett et al. |
| SEQ ID NO: | 7 | aggtgagacc aggcggca | Seq ID No: 3 | of Bennett et al. |
| SEQ ID NO: | 8 | gcagaggcag acgaacca | Seq ID No: 5 | of Bennett et al. |
| SEQ ID NO: | 9 | gcaagcagcc ccagagga | Seq ID No: 6 | of Bennett et al. |
| SEQ ID NO: | 10 | ggtcagcaag cagcccca | Seq ID No.:7 | of Bennett et al. |
| SEQ ID NO: | 11 | gacagcggtc agcaagca | Seq ID No: 8 | of Bennett et al. |
| SEQ ID NO: | 12 | gatggacagc ggtcagca | Seq ID No: 9 | of Bennett et al. |
| SEQ ID NO: | 13 | tctggatgga cagcggtc | Seq ID No.:10 | of Bennett et al. |
| SEQ ID NO: | 14 | ggtggttctg gatggaca | Seq ID No: 11 | of Bennett et al. |
| SEQ ID NO: | 15 | gtgggtggtt ctggatgg | Seq ID No: 12 | of Bennett et al. |
| SEQ ID NO: | 16 | gcagtgggtg gttctgga | Seq ID No: 13 | of Bennett et al. |
| SEQ ID NO: | 17 | ctggcacaaa gaacagca | Seq ID No: 15 | of Bennett et al. |
| SEQ ID NO: | 18 | gtgcagtcac tcaccagt | Seq ID No: 20 | of Bennett et al. |
| SEQ ID NO: | 19 | attccgtttc agtgaact | Seq ID No: 23 | of Bennett et al. |
| SEQ ID NO: | 20 | ttcaccgcaa ggaaggca | Seq ID No: 25 | of Bennett et al. |
| SEQ ID NO: | 21 | ctctgttcca ggtgtcta | Seq ID No: 26 | of Bennett et al. |
| SEQ ID NO: | 22 | ctggtggcag tgtgtctc | Seq ID No: 27 | of Bennett et al. |
| SEQ ID NO: | 23 | ggtgcccttc tgctggac | Seq ID No: 31 | of Bennett et al. |
| SEQ ID NO: | 24 | ctgaggtgcc cttctgct | Seq ID No: 32 | of Bennett et al. |
| SEQ ID NO: | 25 | gtgtctgttt ctgaggtg | Seq ID No: 33 | of Bennett et al. |
| SEQ ID NO: | 26 | acaggtgcag atggtgtc | Seq ID No: 35 | of Bennett et al. |
| SEQ ID NO: | 27 | gtgccagcct tcttcaca | Seq ID No: 37 | of Bennett et al. |
| SEQ ID NO: | 28 | tgcaggacac agctctca | Seq ID No: 40 | of Bennett et al. |
| SEQ ID NO: | 29 | gagcggtgca ggacacag | Seq ID No: 41 | of Bennett et al. |
| SEQ ID NO: | 30 | aatctgcttg accccaaa | Seq ID No: 43 | of Bennett et al. |
| SEQ ID NO: | 31 | gctcgcagat ggtatcag | Seq ID No: 46 | of Bennett et al. |
| SEQ ID NO: | 32 | gcagggctcg cagatggt | Seq ID No: 47 | of Bennett et al. |

| | | | | |
|---|---|---|---|---|
| SEQ ID NO: | 33 | gactgggcag ggctcgca | Seq ID No: 49 | of Bennett et al. |
| SEQ ID NO: | 34 | gcagatgaca cattggag | Seq ID No: 52 | of Bennett et al. |
| SEQ ID NO: | 35 | tcgaaagcag atgacaca | Seq ID No: 53 | of Bennett et al. |
| SEQ ID NO: | 36 | gtccaagggt gacatttt | Seq ID No: 54 | of Bennett et al. |
| SEQ ID NO: | 37 | caggtctttg gtctcaca | Seq ID No: 57 | of Bennett et al. |
| SEQ ID NO: | 38 | ctgttgcaca accaggtc | Seq ID No: 58 | of Bennett et al. |
| SEQ ID NO: | 39 | gtttgtgcct gcctgttg | Seq ID No: 59 | of Bennett et al. |
| SEQ ID NO: | 40 | gtcttgtttg tgcctgcc | Seq ID No: 60 | of Bennett et al. |
| SEQ ID NO: | 41 | caccaccagg gctctcag | Seq ID No: 64 | of Bennett et al. |
| SEQ ID NO: | 42 | gggatcacca ccagggct | Seq ID No: 65 | of Bennett et al. |
| SEQ ID NO: | 43 | gtcgggaaaa ttgatctc | Seq ID No: 71 | of Bennett et al. |
| SEQ ID NO: | 44 | ggagccagga agatcgtc | Seq ID No: 73 | of Bennett et al. |
| SEQ ID NO: | 45 | tggagccagg aagatcgt | Seq ID No: 74 | of Bennett et al. |
| SEQ ID NO: | 46 | tggcatccat gtaaagtc | Seq ID No: 77 | of Bennett et al. |
| SEQ ID NO: | 47 | ggtgcagcct cactgtct | Seq ID No: 81 | of Bennett et al. |
| SEQ ID NO: | 48 | aactgcctgt ttgcccac | Seq ID No: 82 | of Bennett et al. |

The following siRNA sequences are suitable for use in the present invention. (See, e.g., Pluvinet, et al., *Blood*, 2004, 104(12), 3642-3646), the contents of which are incorporated by reference herein.

(SEQ ID NO:49):
5_-GCGAAUUCCUAGACACCUGUU-3_ (siRNA-2 of Pluvinet et al.)
3_-UUCGCUUAAGGAUCUGUGGAC-5_

(SEQ ID NO:50):
5_-CUGGUGAGUGACUGCACAGUU-3_ (siRNA-6 of Pluvinet et al.)
3_-UUGACCACUCACUGACGUGUC-5_

(SEQ ID NO:51):
5_-UACUGCGACCCCAACCUAGUU-3_ (siRNA-8 of Pluvinet et al.)
3_-UUAUGACGCUGGGGUUGGAUC-5_

All siRNA contain a 2 nucleotide overhang at 3'ends.

Oligonucleotides against murine CD40

Examples of murine antisense CD40 oligonucleotides are presented below. Further sequence information is found in published patent application number US 2004/0186071 to Bennett, et al., the contents of which are hereby incorporated by reference herein. The SEQ ID NOs referred to by Bennett, et al. are provided to the right.

Murine

| | | | | |
|---|---|---|---|---|
| SEQ ID NO: | 52 | agacaccatc gcag | Seq. ID No. 116 | of Bennett et al. |
| SEQ ID NO: | 53 | gcgagatcag aagag | Seq. ID No. 117 | of Bennett et al. |

| | | | | |
|---|---|---|---|---|
| SEQ ID NO: | 54 | cgctgtcaac aagca | Seq. ID No. 118 | of Bennett et al. |
| SEQ ID NO: | 55 | ctgccctaga tggac | Seq. ID No. 119 | of Bennett et al. |
| SEQ ID NO: | 56 | ctggctggca caaat | Seq. ID No. 120 | of Bennett et al. |
| SEQ ID NO: | 57 | cttgtccagg gataa | Seq. ID No. 123 | of Bennett et al. |
| SEQ ID NO: | 58 | cacagatgac attag | Seq. ID No. 124 | of Bennett et al. |
| SEQ ID NO: | 59 | tgatatagag aaaca | Seq. ID No. 125 | of Bennett et al. |
| SEQ ID NO: | 60 | ctcattatcc tttgg | Seq. ID No. 127 | of Bennett et al. |
| SEQ ID NO: | 61 | ggttcagacc agg | Seq. ID No. 128 | of Bennett et al. |
| SEQ ID NO: | 62 | tttatttagc cagta | Seq. ID No. 130 | of Bennett et al. |
| SEQ ID NO: | 63 | agccccacgc actgg | Seq. ID No. 131 | of Bennett et al. |
| SEQ ID NO: | 64 | tctcactcct atcccagt | Seq. ID No. 134 | of Bennett et al. |
| SEQ ID NO: | 65 | attagtctga ctcgt | Seq. ID No. 138 | of Bennett et al. |
| SEQ ID NO: | 66 | acattagtct gactc | Seq. ID No. 139 | of Bennett et al. |
| SEQ ID NO: | 67 | cagatgacat tagtc | Seq. ID No. 142 | of Bennett et al. |
| SEQ ID NO: | 68 | ctggactcac cacag | Seq. ID No. 143 | of Bennett et al. |
| SEQ ID NO: | 69 | ggactcacca cagat | Seq. ID No. 144 | of Bennett et al. |
| SEQ ID NO: | 70 | actcaccaca gatga | Seq. ID No. 145 | of Bennett et al. |
| SEQ ID NO: | 71 | tcaccacaga tgaca | Seq. ID No. 146 | of Bennett et al. |
| SEQ ID NO: | 72 | accacagatg acatt | Seq. ID No. 147 | of Bennett et al. |
| SEQ ID NO: | 73 | agatgacatt ag | Seq. ID No. 153 | of Bennett et al. |
| SEQ ID NO: | 74 | cagatgacat tag | Seq. ID No. 154 | of Bennett et al. |
| SEQ ID NO: | 75 | acagatgaca ttag | Seq. ID No. 155 | of Bennett et al. |
| SEQ ID NO: | 76 | ccacagatga cattag | Seq. ID No. 156 | of Bennett et al. |
| SEQ ID NO: | 77 | accacagatg acattag | Seq. ID No. 157 | of Bennett et al. |
| SEQ ID NO: | 78 | caccacagat gacattag | Seq. ID No. 158 | of Bennett et al. |
| SEQ ID NO: | 79 | tcaccacaga tgacattag | Seq. ID No. 159 | of Bennett et al. |
| SEQ ID NO: | 80 | ctcaccacag atgacattag | Seq. ID No. 160 | of Bennett et al. |

Oligonucleotides against rat CD40

Examples of rat antisense CD40 oligonucleotides are presented below. (See, Gao, Ph.D. thesis, 2003, University of Göttingen, Germany).

| | | |
|---|---|---|
| SEQ ID NO:81 | accgctgtcaacaagcagc | (rAS2 of Gao) |
| SEQ ID NO:82 | tcctagatggaccgctgt | (rAS3 of Gao) |
| SEQ ID NO:83 | taacacactgtcctag | (rAS4 of Gao) |

Oligonucleotides against porcine CD40

Examples of porcine antisense CD40 oligonucleotides are presented below. See, Rushworth, et al., *Transplantation,* 2002, 73(4), 635-642, the contents of which are incorporated by reference herein.

| | | |
|---|---|---|
| SEQ ID NO:84 | gctgatgacagtgtttct | (Aso3 of Rushworth et al.) |
| SEQ ID NO:85 | gcctcactctcgctcctg | (Aso8 of Rushworth et al.) |
| SEQ ID NO:86 | ggactgtatctggactgc | (Aso9 of Rushworth et al.) |
| SEQ ID NO:87 | gtggacagtcatgtatat | (Aso10 of Rushworth et al.) |

Figure legends

| | | |
|---|---|---|
| **Figure 1**: | Microscopic scoring of colonic damage. | |
| | Controlcontrol animals, PBS treated | |
| | CD40/ 0 | treated at day0, 4h prior induction |
| | CD40/0_3 | treated at day0, 4k prior induction and day3 |
| | SCR/ 0 | treated with scrambled control, 4h prior induction |
| | CD40/ 3 | treated at day 3 only |
| | SCR/ 3 | treated with scrambled control at day 3 |
| **Figure 2:** | Colon sections after various treatments. | |
| | A | normal, unaffected bowel wall |
| | B | inflamed, but untreated bowel wall |
| | C | treatmant prior colitis induction using the scrambled control |
| | D | treatment prior colitis induction using the specific CD40 antisense |

**Figure 3**: Perfusion index (filled columns) and stasis index (open columns) in allogeneic control (control), CD40 antisense ODN (AS) or scrambled control ODN (SCR) treated grafts 7 days post transplantation (n=3). * p<0.05 vs. control, # p<0.05 AS vs. SCR

**Figure 4**: Functional capillary density (FCD) in the mucosa in allogeneic control (control), CD40 antisense ODN (AS) or scrambled control ODN (SCR) treated grafts 7 days post transplantation (n=3). * p<0.05 vs. control, # p<0.05 AS vs. SCR

**Figure 5**: Red blood cell velocity (RBCV) in submucosal vessels of small bowel transplants in allogeneic control (control), CD40 antisense ODN (AS) or scrambled control ODN (SCR) treated grafts 7 days post transplantation (n=3). * p<0.05 vs. control, # p<0.05 AS vs. SCR

**Figure 6:** Leukocyte-endothelial cell interaction in allogeneic control (control), CD40 antisense ODN (AS) or scrambled control ODN (SCR) treated grafts 7 days post transplantation (n=3). * p<0.05 vs. control, # p<0.05 AS vs. SCR

**Figure 7:** Porcine CD40 cDNA sequence (SEQ ID NO:4) for targeting in accordance with the present invention.

## Claims

1. A pharmaceutical composition comprising an oligonucleotide as an active agent, which oligonucleotide is adapted to target nucleic acids encoding CD40 thereby to modulate the expression of CD40 in mammalian cells, and a liposome as an excipient; **characterised in that** said liposome is an amphoteric liposome, and a pharmaceutically acceptable carrier.

2. A pharmaceutical composition as claimed in claim 1, **characterised in that** said liposome has an isoelectric point of between 4 and 7.4.

3. A pharmaceutical composition as claimed in claim 1 or claim 2, **characterised in that** said amphoteric liposome is negatively charged or neutral at pH 7.4 and cationic at pH 4.

4. A pharmaceutical composition as claimed in claim 1, claim 2 or claim 3, **characterised in that** said amphoteric liposome is formed from a lipid phase comprising an amphoteric lipid and a neutral phospholipid.

5. A pharmaceutical composition as claimed in claim 4, **characterised in that** said lipid phase comprises 5 to 30 mol. % of said amphoteric lipid.

6. A pharmaceutical composition as claimed in claim 4 or claim 5, **characterised in that** said amphoteric lipid is selected from the group consisting of HistChol, HistDG, isoHistSuccDG, Acylcarnosin and HCCHol.

7. Pharmaceutical composition of claim 4, **characterised in that** said amphoteric lipid is selected from the group of HistChol.

8. A pharmaceutical composition as claimed in claim 1, claim 2 or claim 3, **characterised in that** said amphoteric liposome is formed from a lipid phase comprising a mixture of lipid components with amphoteric properties and a neutral phospholipid.

9. A pharmaceutical composition as claimed in claim 8, **characterised in that** said mixture of lipid components comprises anionic or cationic components, wherein at least one such component is pH responsive.

10. A pharmaceutical composition as claimed in claim 9, **characterised in that** said lipid components comprise one or more anionic lipids selected from the group consisting of DGSucc, DMPS, DPPS, DOPS, POPS, DMPG, DPPG, DOPG, POPG, DMPA, DPPA, DOPA, POPA, CHEMS and Cetyl-P.

11. A pharmaceutical composition as claimed in claim 10, **characterised in that** said lipid components comprise one or more anionic lipids selected from the group consisting of DGSucc, DOPA, CHEMS and Cetyl-P.

12. A pharmaceutical composition as claimed in any of claims 8 to 11, **characterised in that** said lipid components comprise one or more cationic lipids selected from the group consisting of DMTAP, DPTAP, DOTAP,DC-Chol, MoChol, HisChol, DPIM, CHIM, DORIE, DDAB,DAC-Chol, TC-Chol, DOTMA, DOGS, (C18)2Gly+ N,N-dioctadecylamido-glycin, CTAP, CPyC, DODAP and DOEPC.

13. A pharmaceutical composition as claimed in any of claims 8 to 12, **characterised in that** said lipid components comprise one or more cationic lipids selected from the group consisting of DOTAP, DC-Chol, MoChol and HisChol.

14. A pharmaceutical composition as claimed in any of claims 4 to 13, **characterised in that** said neutral phospholipid includes a phosphatidylcholine and represents at least 20 mol% of the total lipids.

**15.** A pharmaceutical composition as claimed in claim 14, **characterised in that** said neutral phosphatidylcholine is selected from the group consisting of POPC, natural or hydrogenated soy bean PC, natural or hydrogenated egg PC, DMPC, DPPC, DSPC and DOPC.

**16.** A pharmaceutical composition as claimed in claim 14, **characterised in that** said phosphatidylcholine comprises POPC, non-hydrogenated soy bean PC or non-hydrogenated egg PC.

**17.** Pharmaceutical composition of claim 4-13, **characterised in that** said neutral phospholipid comprises phosphatidylcholine or phosphatidylethanolamine or a mixture of both and represents at least 20mol% of the total lipids.

**18.** A pharmaceutical composition as claimed in claim 17, **characterised in that** said phosphatidylethanolamine is selected from DOPE, DMPE and DPPE and said phosphatidylcholine is selected from the group consisting of POPC, natural or hydrogenated soy bean PC, natural or hydrogenated egg PC, DMPC, DPPC, DSPC and DOPC.

**19.** A pharmaceutical composition as claimed in claim16, **characterised in that** said lipid phase comprises about 60 mol.% POPC, about 10 mol.% DOTAP and about 30 mol.% CHEMS.

**20.** A pharmaceutical composition as claimed in claim 14, **characterised in that** said lipid phase further comprises cholesterol.

**21.** A pharmaceutical composition as claimed in claim 20, **characterised in that** said lipid phase comprises about 60 mol.% POPC, about 20 mol.% HistChol and about 20 mol.% Chol.

**22.** A pharmaceutical composition as claimed in any of claims 1 to 21, wherein said composition further comprises a vehicle and is formulated for local administration.

**23.** A pharmaceutical composition as claimed in any preceding claim, **characterised in that** said liposome has a size in the range 50 and 500nm and, more preferentially having a size between 150 and 300nm.

**24.** A pharmaceutical composition as claimed in any preceding claim, **characterised in that** said oligonucleotide is an antisense oligonucleotide of 15 to 50 basepairs length.

**25.** A pharmaceutical composition as claimed in claim 24, **characterised in that** said oligonucleotide contains phosphothioate linkages, 2'MOE modified nucleobases, LNA nucleobases, FANA nucleobases or naturally occurring ribonucleotides or deoxyribonucleotides.

**26.** A pharmaceutical composition as claimed in any of claims 1 to 23 **characterised in that** the oligonucleotide is a siRNA of 15 to 50 basepairs length.

**27.** A pharmaceutical composition as claimed in preceding claim, **characterised in that** the oligonucleotide targets the human CD40 gene.

**28.** Use of a pharmaceutical composition as claimed in any preceding claim for the prevention or treatment of an inflammatory, immune or autoimmune disorder of a human or non-human animal.

**29.** Use of a pharmaceutical composition as claimed in claim 22 for the prevention or treatment of graft rejection, graft-versus-host disease, inflammatory bowel disease, Morbus Crohn, Colitis ulcerosa, Asthma bronchiale, COPD, atopic dermatitis, psoriasis and allergic rhinitis.

Figure 1:

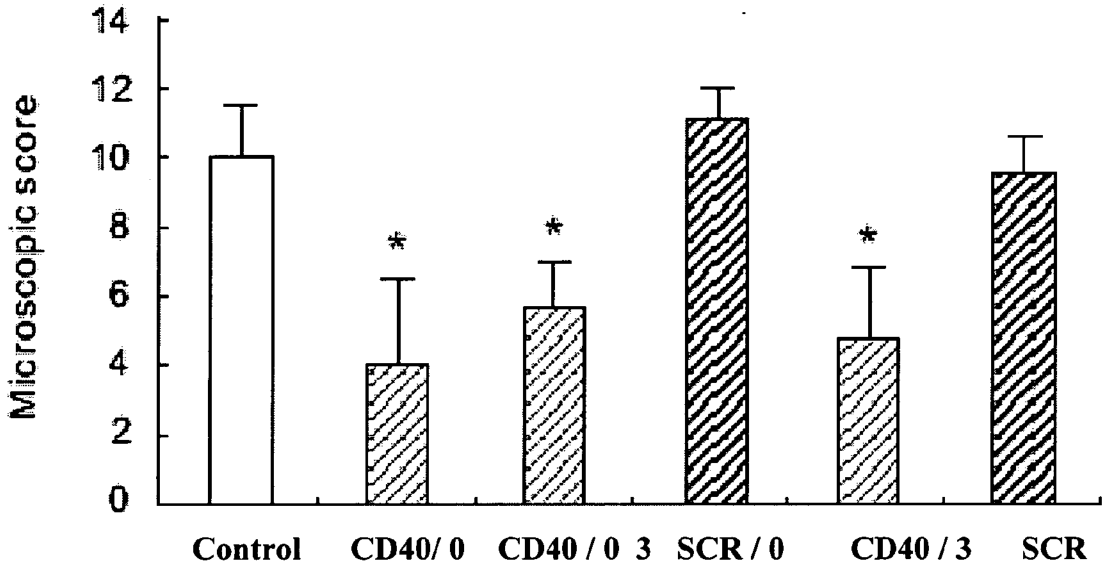
Microscopic score
14
12
10
8
6
4
2
0
*
*
*
Control
CD40/ 0
CD40 / 0 3
SCR / 0
CD40 / 3
SCR

Figure 2:

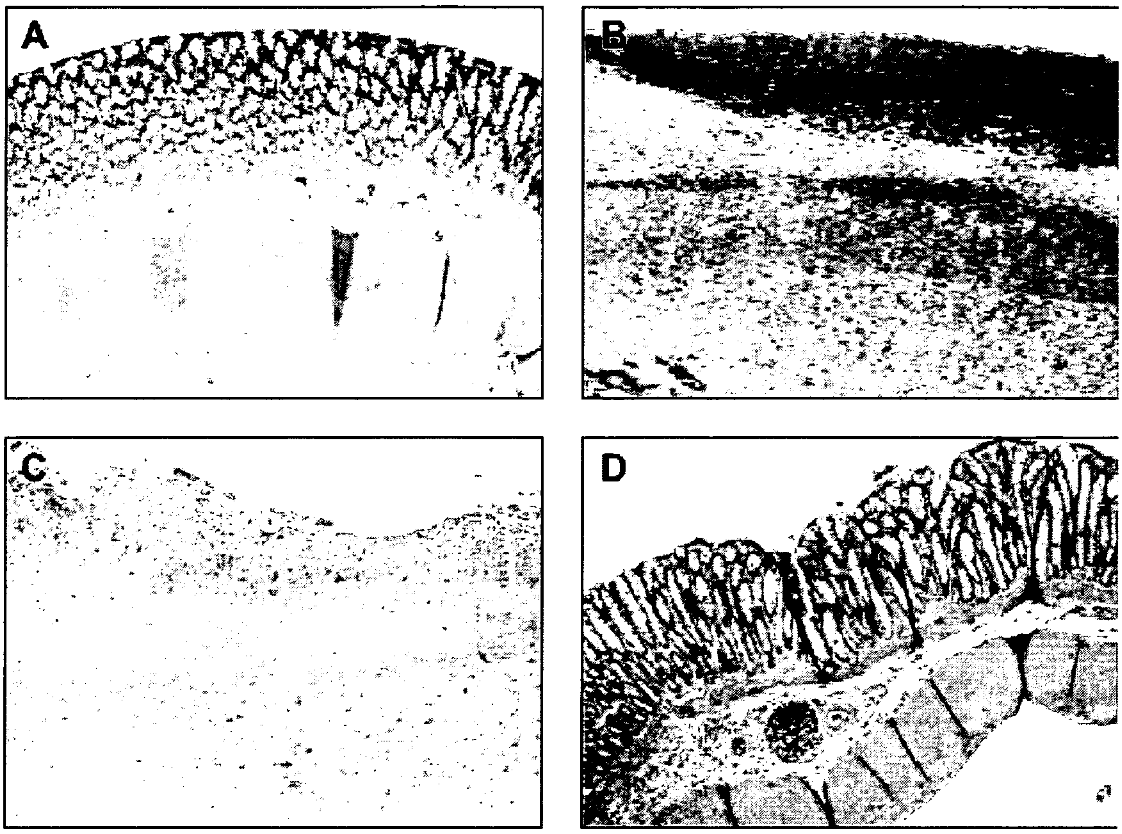

Figure 3:

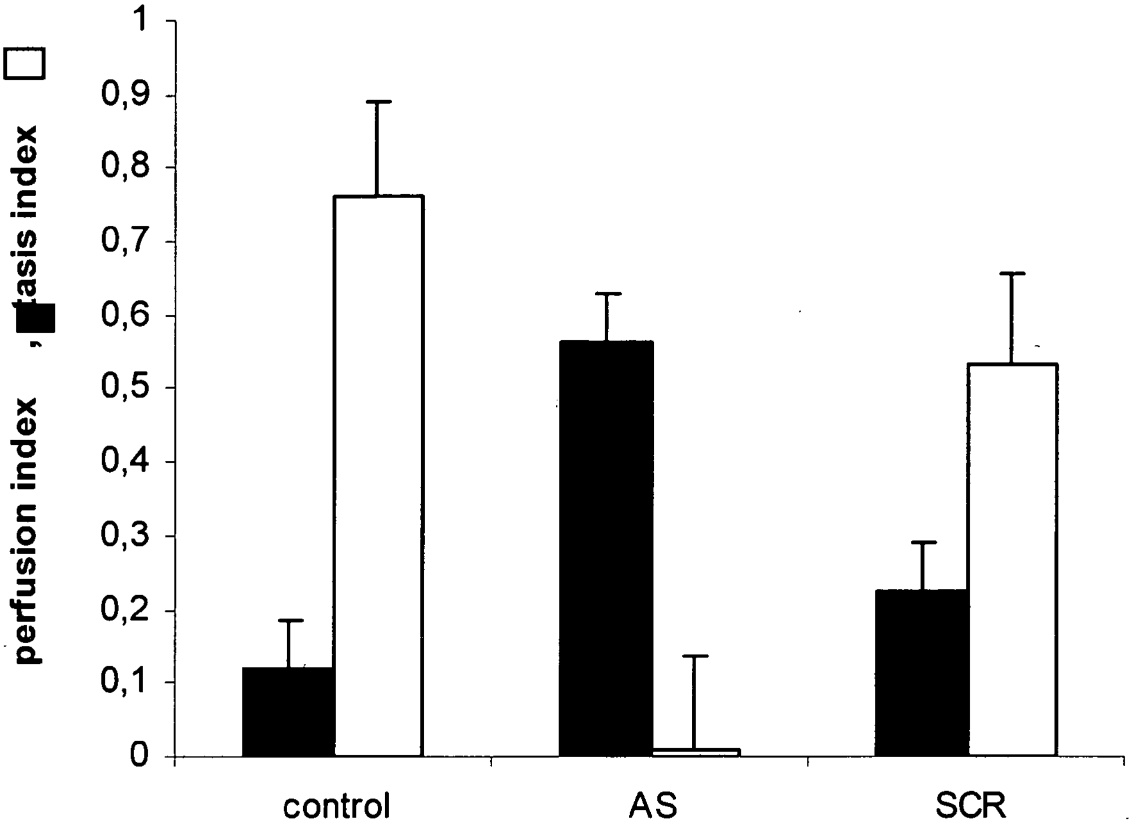
perfusion index ■, stasis index □
1
0,9
0,8
0,7
0,6
0,5
0,4
0,3
0,2
0,1
0
control
AS
SCR

Figure 4:

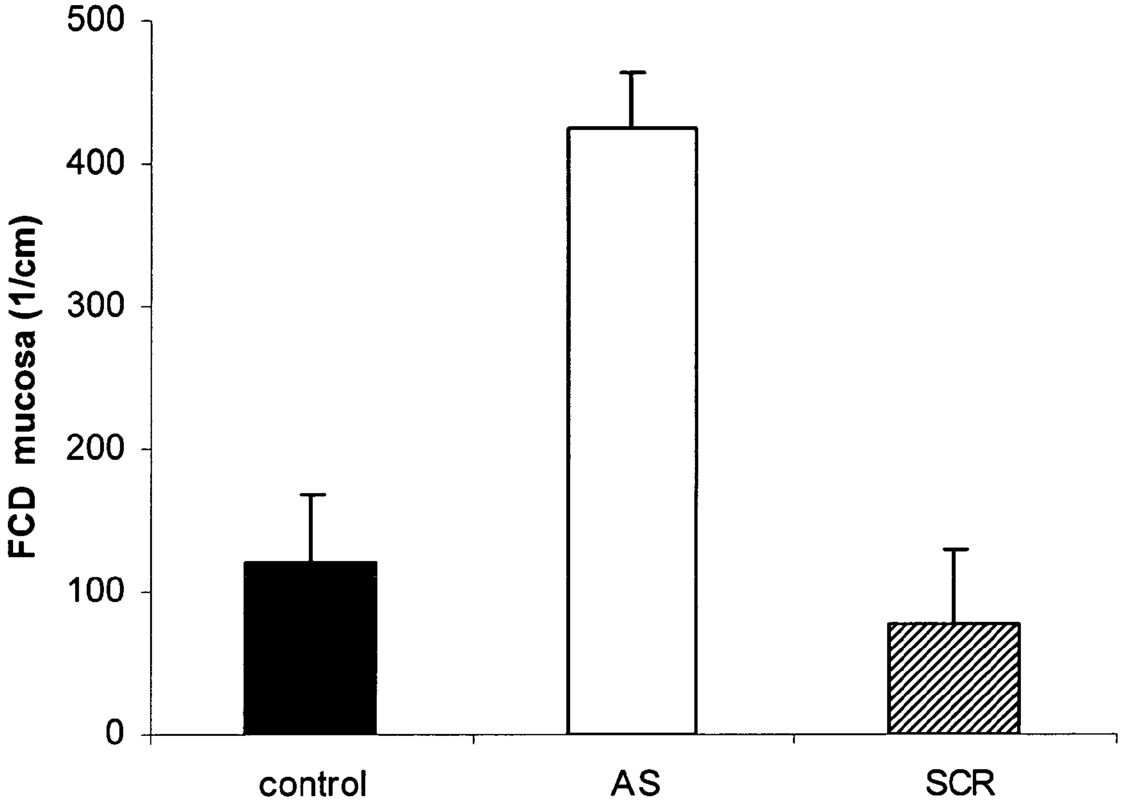

Figure 5:

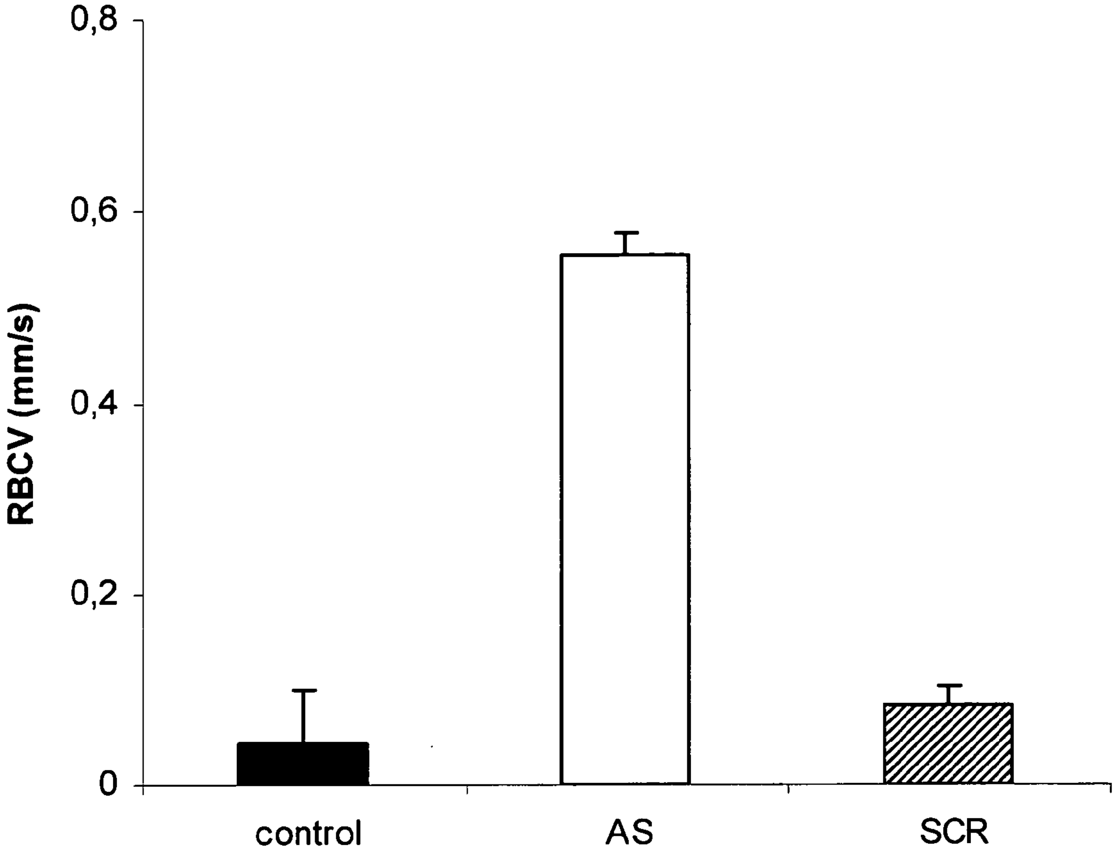

**Figure 6:**

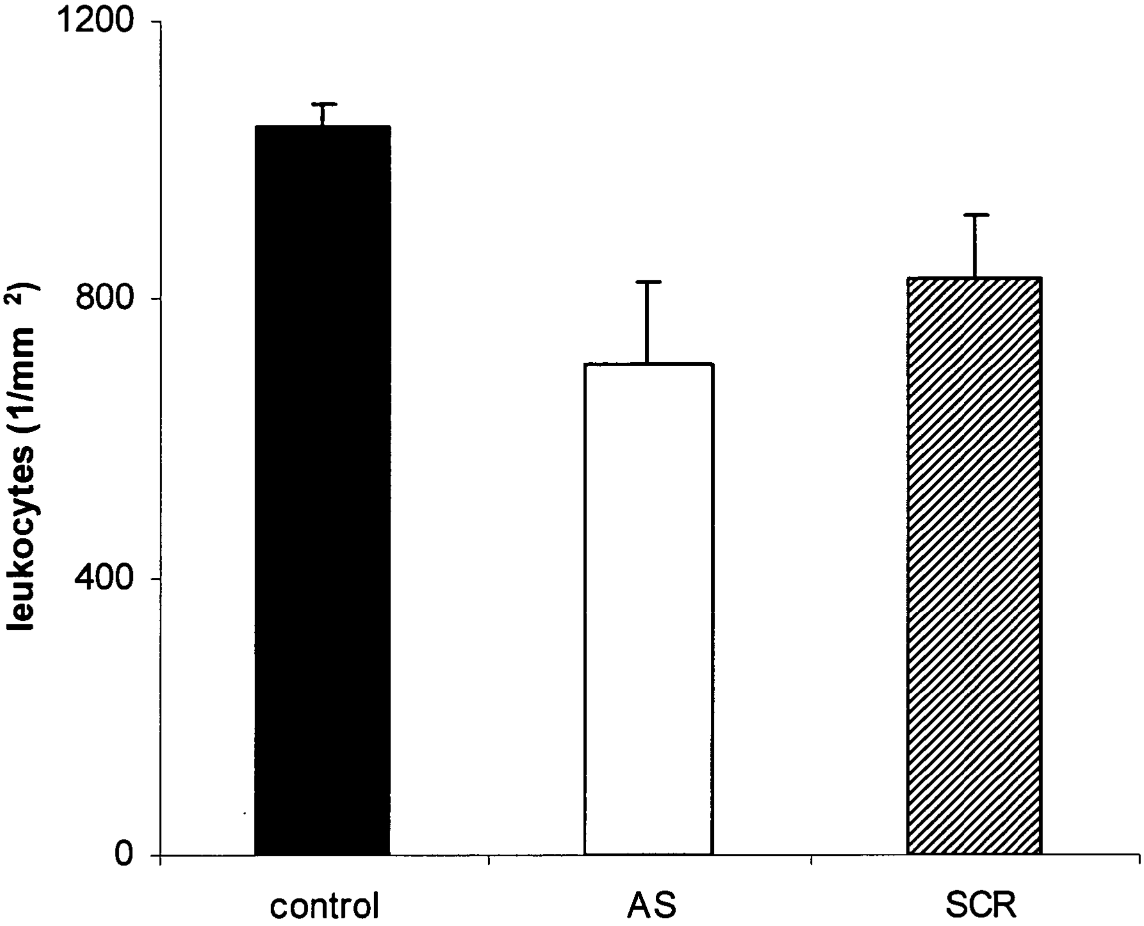

Figure 7:

(SEQ ID NO:4)

gcctcgccATGGTTCGTCTGCCTCTGAAGTGTCTCCTCTGGGGCTGCTTTTTGACCGCCGTCCACCCAGAACCACCCACTTCATGCAAAGAAAACCAATACCCAACAAACAGCCGG
ASO 1

TGCTGTAATTTCTGCCCGGCCAGGACAGAAACTGGTGAACCACTGCACAGAGGTCACTGAAACAGAATGCCTTCCTTGCAGTTCCAGCCAATTCCTAGCCACCTGGAATAGAGAGAA
ASO 2

ACACTGTCATCAGCACAAATACTGCGACCCCAACCTAGGTCTCCAGGTCCAGAGGGAGGGCACCTCGAAAACAGACACCACTTGTGTGTGCAGTGAAGGCCATCACTGTACCAACA
ASO 3

GCGCCTGTGAAAGTTGCACCTTGCACAGCTTGTGCTTCCCTGGCCTCGGGGTCAAGCACATGGCGACAGAGGTTTCTGACACTATCTGTGAACCCTGCCCAGTTGGCTTCTTCTCC
ASO 4

AATGTATCATCTGCTTCAGAAAAGTGTCAGCCTTGCACAAGCTGCGAGAGCAAAGGCCTGGTGGAACAACGTGCGGGGACTAACAAGACCGATGTTGTCTGTGGTTTCCAGAGTCG
ASO 5

GATGAGAGCCCTGGTGGTTATCCCCATCAGGCTGGGGATCCTGTTTGCCGTCCTGTTGGTATTTCTCTGTATCAGAAAGGTGACCAAGGAGCAGGAGACTAAGGCCCTGCACCCTA
ASO 6

AGACTGAAAGGCAGGATCCCGTGGAGACGATTGATCTGGAGGATTTTCCCGACTCCACCGCTCCGGTGCAGGAGACCTTACATTGGTGCCAGCCCGTCACCCAGGAGGATGGCAAA
ASO 7

GAGAGCCGCATCTCGGTGCAGGAGCGACAGTGAggctgtgcgtggccaggagcgtggaggcacgggcacaggggcatgtgactggagagcccggggcggctgctgctgctgtggcg
ASO 8

gtggtgagagggtggtgctgggcacagccccttctgcctgcaccccctgcagtccagatacagtccacctcgaggagcttctcaccccagccctggagcccattcaatctcagtttg
ASO 9

cttttaaagatggagacaaactttggggagtcacagctacagtaataaccaccagagcttccaacccagaggttcagtacctgcagatgcaagggatggcgtctaggagcccagg

aggcatatacatgactgtccaccactgcattgttcgtgacagtgagtgactggaaactgcttaactgtcccatcaacagggactggctaaataaaattgtaacatgtttatgcaaa
ASO 10

aaaaaaaa

European Patent Office

# PARTIAL EUROPEAN SEARCH REPORT

which under Rule 45 of the European Patent Convention shall be considered, for the purposes of subsequent proceedings, as the European search report

Application Number
EP 05 09 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/099697 A1 (PANZNER STEFFEN ET AL) 29 May 2003 (2003-05-29)<br>* page 1, paragraph 15 *<br>* page 3, paragraphs 65,66,71-73 *<br>* page 5; examples 1,2 *<br>* page 6 - page 7; examples 6-8 * | 1-29 | A61K9/127<br>A61P1/00<br>A61P37/06 |
| D,X | & WO 02/066012 A (PANZNER, STEFFEN ET AL) 29 August 2002 (2002-08-29)<br>----- | 1-29 | |
| X | US 2004/037874 A1 (HONG KEELUNG ET AL) 26 February 2004 (2004-02-26)<br>* page 3, paragraphs 31-33,37,38 *<br>* page 6, paragraph 59 *<br>* page 9, paragraph 71-73 *<br>* page 12, paragraph 94 *<br>* page 17; table 8 *<br>* page 20 - page 21; examples 19-22 *<br>-----<br>-/-- | 1-5, 8-20, 22-29 | |

TECHNICAL FIELDS SEARCHED (IPC)

A61K
A61P

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 9 March 2006 | Gomez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C07)

European Patent Office

# PARTIAL EUROPEAN SEARCH REPORT

Application Number
EP 05 09 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAFEZ I M ET AL: "On the mechanism whereby cationic lipids promote intracellular delivery of polynucleic acids" GENE THERAPY, MACMILLAN PRESS LTD., BASINGSTOKE, GB, vol. 8, 2001, pages 1188-1196, XP002358998 ISSN: 0969-7128 * abstract * * page 1188, right-hand column, last paragraph - page 1190, left-hand column, paragraph 2 * * page 1191, left-hand column, last paragraph - right-hand column, paragraph 1; table 1 * * page 1194, left-hand column, paragraph 3 * | 1-5, 8-20, 22-29 | |
| | ----- | | TECHNICAL FIELDS SEARCHED (IPC) |
| X | WROBEL I ET AL: "FUSION OF CATIONIC LIPOSOMES WITH MAMMALIAN CELLS OCCURS AFTER ENDOCYTOSIS" BIOCHIMICA ET BIOPHYSICA ACTA. BIOMEMBRANES, AMSTERDAM, NL, vol. 1235, no. 2, 4 May 1995 (1995-05-04), pages 296-304, XP000619562 ISSN: 0005-2736 * abstract * * page 296, right-hand column, last paragraph - page 297, left-hand column, paragraph 1 * * page 298, right-hand column, paragraph 2 - page 299, right-hand column, paragraph 1 * * page 303, right-hand column, paragraph 2 * | 1-5, 8-20, 22-29 | |
| | ----- | | |
| | -/-- | | |

2

EPO FORM 1503 03.82 (P04C10)

European Patent Office

# PARTIAL EUROPEAN SEARCH REPORT

Application Number
EP 05 09 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 096 335 A (THIERRY ET AL) 1 August 2000 (2000-08-01)<br>* column 3, line 51 - column 4, line 40 *<br>* column 5, lines 3-67 *<br>* column 6, lines 35-67 *<br>* column 7, lines 17-20 *<br>* column 16; example 10 *<br>----- | 1-5, 8-20, 22-29 | |
| X | US 2004/120997 A1 (PANZNER STEFFEN ET AL) 24 June 2004 (2004-06-24)<br>* page 1, paragraphs 13,14 *<br>* page 4, paragraph 45 - page 5, paragraph 48 *<br>* page 6 - page 7; examples 1-4 *<br>* claims 1-21 * | 1-7, 14-18, 22-29 | |
| D,X | & WO 02/066489 A (PANZNER, STEFFEN ET AL) 29 August 2002 (2002-08-29)<br>----- | 1-7, 14-18, 22-29 | |
| P,X, L | WO 2005/094783 A (ENDERT, GEROLD ET AL) 13 October 2005 (2005-10-13)<br>* page 3, line 3 - page 4, line 15 *<br>* page 5, lines 1-19 *<br>* page 7 - page 8; tables 1,3 *<br>* page 11 - page 12; example 2 *<br>* page 13 - page 14; example 5 *<br>----- | 1-29 | |

TECHNICAL FIELDS SEARCHED (IPC)

2

EPO FORM 1503 03.82 (P04C10)

European Patent Office

**INCOMPLETE SEARCH SHEET C**

Application Number

EP 05 09 0322

Although claims 28 and 29 are directed to a method of treatment of the human/animal body (Article 52(4) EPC), the search has been carried out and based on the alleged effects of the composition.

-----

**ANNEX TO THE EUROPEAN SEARCH REPORT ON EUROPEAN PATENT APPLICATION NO.** EP 05 09 0322

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2006

| Patent document cited in search report | Publication date | Patent family member(s) | Publication date |
|---|---|---|---|
| US 2003099697 A1 | 29-05-2003 | BR 0207775 A | 30-03-2004 |
| | | CA 2438116 A1 | 29-08-2002 |
| | | CN 1492756 A | 28-04-2004 |
| | | DE 10109897 A1 | 07-11-2002 |
| | | WO 02066012 A2 | 29-08-2002 |
| | | EP 1363601 A2 | 26-11-2003 |
| | | JP 2004525898 T | 26-08-2004 |
| WO 02066012 A | 29-08-2002 | BR 0207775 A | 30-03-2004 |
| | | CA 2438116 A1 | 29-08-2002 |
| | | CN 1492756 A | 28-04-2004 |
| | | DE 10109897 A1 | 07-11-2002 |
| | | EP 1363601 A2 | 26-11-2003 |
| | | JP 2004525898 T | 26-08-2004 |
| | | US 2003099697 A1 | 29-05-2003 |
| US 2004037874 A1 | 26-02-2004 | NONE | |
| US 6096335 A | 01-08-2000 | AT 276742 T | 15-10-2004 |
| | | AU 9260898 A | 22-02-1999 |
| | | CA 2296394 A1 | 11-02-1999 |
| | | DE 69826488 D1 | 28-10-2004 |
| | | DE 69826488 T2 | 01-12-2005 |
| | | WO 9906026 A1 | 11-02-1999 |
| | | EP 1001750 A1 | 24-05-2000 |
| | | ES 2229534 T3 | 16-04-2005 |
| | | FR 2766706 A1 | 05-02-1999 |
| | | JP 2001511440 T | 14-08-2001 |
| US 2004120997 A1 | 24-06-2004 | BR 0207776 A | 23-03-2004 |
| | | CA 2438910 A1 | 29-08-2002 |
| | | CN 1492876 A | 28-04-2004 |
| | | DE 10109898 A1 | 05-09-2002 |
| | | WO 02066489 A2 | 29-08-2002 |
| | | WO 02066490 A2 | 29-08-2002 |
| | | EP 1363933 A2 | 26-11-2003 |
| | | EP 1363932 A2 | 26-11-2003 |
| | | JP 2004521917 T | 22-07-2004 |
| | | JP 2004525902 T | 26-08-2004 |
| | | US 2004131666 A1 | 08-07-2004 |
| WO 02066489 A | 29-08-2002 | BR 0207776 A | 23-03-2004 |
| | | CA 2438910 A1 | 29-08-2002 |
| | | CN 1492876 A | 28-04-2004 |
| | | DE 10109898 A1 | 05-09-2002 |
| | | WO 02066490 A2 | 29-08-2002 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 09 0322

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2006

| Patent document cited in search report | Publication date | Patent family member(s) | Publication date |
|---|---|---|---|
| WO 02066489 A | | EP 1363933 A2 | 26-11-2003 |
| | | EP 1363932 A2 | 26-11-2003 |
| | | JP 2004521917 T | 22-07-2004 |
| | | JP 2004525902 T | 26-08-2004 |
| | | US 2004131666 A1 | 08-07-2004 |
| | | US 2004120997 A1 | 24-06-2004 |
| WO 2005094783 A | 13-10-2005 | NONE | |

EPO FORM P0459

For more details about this annex : see Official Journal of the European Patent Office, No. 12/82